# EUROPEAN PATENT APPLICATION

(11) **EP 2 853 896 A2**
(43) Date of publication of application: **01.04.2015**
(21) Application number: 14186939.6
(22) Date of filing: 30.09.2014
(51) Int. Cl.: G01N 33/68

(54) **A method and apparatus for obtaining an information related to eosinophilic airway inflammation**

(30) Priority: 30.09.2013 JP 2013204787
(71) Applicant: SYSMEX CORPORATION, Kobe-shi, Hyogo 651-0073 (JP)
(72) Inventor: Hasegawa, Takehiro, Kobe-shi, Hyogo 651-0073 (JP)
(74) Representative: Paemen, Liesbet R.J.

(57) **Abstract**

The present invention provides a method of obtaining an information related to eosinophilic airway inflammation in a subject. The method comprises steps of: measuring an amount of at least one marker selected from CXCL9 and CCL27 in a blood sample in a subject; and obtaining an information related to eosinophilic airway inflammation in the subject based on the resulting measured value.

## Description

### THCHNICAL FIELD

The present invention is related to a method and an apparatus for obtaining an information related to eosinophilic airway inflammation.

### BACKGROUND

Bronchial asthma (hereinafter simply referred to as "asthma") is characterized by chronic inflammation of the airway, various levels of reversible airway obstruction, progress of bronchial hyper-responsiveness, clinical recurrent cough, wheeze, and dyspnea. The important points in the differential diagnosis of asthma are as follows: (1) paroxysmal cough, wheeze, recurrent dyspnea; (2) reversible airflow limitation; and (3) non-association with other cardiopulmonary diseases. The therapy of asthma controls the frequency of attacks of asthma in patients, and aims at allowing the patients to perform the same daily activities as healthy individuals. In the current guidelines, the therapeutic strategy is determined by monitoring symptoms of asthma and respiratory function.

When the presence of airway inflammation is observed with the above findings (1) to (3), asthma is indicated. Eosinophils and basophils infiltrated into the airway serve as effector cells, thereby causing airway inflammation. Particularly, the airway inflammation caused by the infiltration of proinflammatory cell population mainly including eosinophils is also referred to as "eosinophilic airway inflammation", and is the most important clinical condition that induces severe attacks of asthma. Examples of indications for determining (monitoring) the status of eosinophilic airway inflammation include high eosinophils in the bronchoalveolar lavage fluid (BALF) and sputum eosinophil count.

A method described in US Patent No. 8,053,199 is one example of methods for discriminating high eosinophils in the bronchoalveolar lavage fluid (BALF). US Patent No. 8,053,199 describes that patients with severe asthma can be distinguished from patients with non-severe asthma based on the patterns of specific cytokine levels in the bronchoalveolar lavage fluid (BALF). US Patent No. 8,053,199 describes that MIG (CXCL9, CXC Chemokine Ligand 9) are cytokines of which the levels are decreased in the patients with severe asthma, and a combination of MIG, Eotaxin, IL-8-(Interleukin-8), and IL-17 (Interleukin-17) levels is useful in discriminating high eosinophils. However, the method for collecting the bronchoalveolar lavage fluid (BALF) is highly invasive.

Further, there is a report that the sputum eosinophil count is useful in discriminating the types of asthma and determining the therapeutic strategy (refer to The Lancet, Volume 360, Pages 1715-1721, 2002 and Am J Respir Crit Care Med, Volume 178, Pages 218-224, 2008).

For example, The Lancet (Volume 360, Pages 1715-1721, 2002) describes that therapeutic management based on sputum eosinophil count significantly reduces acute exacerbation (severe attacks) as compared with the conventional standard therapeutic management.

Further, Am J Respir Crit Care Med (Volume 178, Pages 218-224, 2008) describes that an inflammation predominant group (group with low symptom level and high level of eosinophilic inflammation) can be identified from patients with intractable asthma by using the information of eosinophilic inflammation, and when therapeutic management based on sputum eosinophils is performed on the group, deterioration in asthma can be reduced as compared with the standard therapeutic management.

For the above described reasons, the test for the sputum eosinophil count (sputum test) has been performed in clinical practice. However, it is not possible to obtain spontaneously produced sputum samples from all the patients with asthma in the sputum test. When the spontaneously produced sputum samples are not obtained, a method for allowing a patient to inhale a hypertonic saline solution and to induce sputum production is used. However, there is a possibility that the method may be accompanied by pharyngeal pain or may induce asthmatic by stimuli to the airway.

Therefore, in the search of biomarkers which can provide an indication of eosinophilic airway inflammation, blood attracts attention as a specimen which can be reliably and safely collected from patients with asthma.

For example, J Allergy Clin Immunol, Volume 130, Pages 647-654, 2012 describes that a blood periostin level in patients with severe asthma who present symptoms when treated with inhaled corticosteroid can be used as a systemic biomarker of eosinophilic airway inflammation.

### SUMMARY OF THE INVENTION

The scope of the present invention is defined solely by the appended claims, and is not affected to any degree by the statements within this summary.

The present invention provides a method of obtaining an information related to eosinophilic airway inflammation in a subject. The method comprises steps of: measuring an amount of at least one marker selected from CXCL9 and CCL27 in a blood sample in a subject; and obtaining an information related to eosinophilic airway inflammation in the subject based on the resulting measured value.

The present invention provides an apparatus for obtaining an information related to eosinophilic airway inflammation. The apparatus comprises a measurement unit which obtains an information related to an amount of at least one marker selected from CXCL9 and CCL27 in a blood sample of a subject; an information processing unit which obtains an information related to eosinophilic airway inflammation of the subject based on the information related to the amount of the marker obtained by the measurement unit; and an output unit which outputs the information obtained by the information processing unit.

On the basis of the amount of the marker of the present invention in a blood sample of a subject, information related to eosinophilic airway inflammation in the subject can be obtained.

### BRIEF DESCRIPTION OF THE DRAWINGS

Fig. 1A shows analysis results of the significant test, sensitivity, and specificity of CCL27 obtained by using the serum of 39 patients with asthma (33 sputum eosinophil-positive patients and 6 sputum eosinophil-negative patients);
Fig. 1B shows analysis results of the significant test, sensitivity, and specificity of CXCL9 obtained by using the serum of 39 patients with asthma (33 sputum eosinophil-positive patients and 6 sputum eosinophil-negative patients);
Fig. 1C shows analysis results of the significant test, sensitivity, and specificity of Periostin obtained by using the serum of 39 patients with asthma (33 sputum eosinophil-positive patients and 6 sputum eosinophil-negative patients);
Fig. 1D shows analysis results of the significant test, sensitivity, and specificity of Eotaxin obtained by using the serum of 39 patients with asthma (33 sputum eosinophil-positive patients and 6 sputum eosinophil-negative patients);
Fig. 1E shows analysis results of the significant test, sensitivity, and specificity of TARC obtained by using the serum of 39 patients with asthma (33 sputum eosinophil-positive patients and 6 sputum eosinophil-negative patients);
Fig. 2A shows analysis results of the significant test, sensitivity, and specificity of CCL27 obtained by using the serum of 39 patients with asthma (33 sputum eosinophil-positive patients and 6 sputum eosinophil-negative patients) and the serum of 12 healthy adults without asthma;
Fig. 2B shows analysis results of the significant test, sensitivity, and specificity of CXCL9 obtained by using the serum of 39 patients with asthma (33 sputum eosinophil-positive patients and 6 sputum eosinophil-negative patients) and the serum of 12 healthy adults without asthma;
Fig. 2C shows analysis results of the significant test, sensitivity, and specificity of Periostin obtained by using the serum of 39 patients with asthma (33 sputum eosinophil-positive patients and 6 sputum eosinophil-negative patients) and the serum of 12 healthy adults without asthma;
Fig. 2D shows analysis results of the significant test, sensitivity, and specificity of Eotaxin obtained by using the serum of 39 patients with asthma (33 sputum eosinophil-positive patients and 6 sputum eosinophil-negative patients) and the serum of 12 healthy adults without asthma;
Fig. 2E shows analysis results of the significant test, sensitivity, and specificity of TARC obtained by using the serum of 39 patients with asthma (33 sputum eosinophil-positive patients and 6 sputum eosinophil-negative patients) and the serum of 12 healthy adults without asthma;
Fig. 3A shows analysis results of the significant test, sensitivity, and specificity obtained by the combination of the measured values of CCL27 and CXCL9 produced in Example 1;
Fig. 3B shows analysis results of the significant test, sensitivity, and specificity obtained by combining the measured values of CCL27 and CXCL9 produced in Example 1 with the measured value of IL-25;
Fig. 4 shows a first aspect of an apparatus for obtaining an information related to eosinophilic airway inflammation; and
Fig. 5 is a flow chart showing an example of obtaining the information related to eosinophilic airway inflammation.

### DETAILED DESCRIPTION OF THE PREFERRED EMBODIMENTS

The preferred embodiments of the present invention will be described hereinafter with reference to the drawings.

A first step of the method to obtain an information related to eosinophilic airway inflammation provided herein (hereinafter, also simply referred to as "method") includes measuring the amount of at least one marker selected from CCL27 and CXCL9 in a blood sample of a subject (measuring step).

In the embodiments of the present method, the subject is not particularly limited and examples thereof include patients who have been diagnosed with asthma by doctors, adults or children who are likely affected with asthma, and healthy adults or children who are unaffected with asthma (without asthma).

In the embodiments of the present method, the blood sample is blood (whole blood), serum or plasma. Particularly, it is preferable to use a blood sample which can be simply collected. Examples thereof include peripheral blood and serum and plasma obtained from the peripheral blood.

CCL27 is also referred to as "CTACK", and is well-known to those skilled in the art as a chemokine which is specifically expressed on epidermal keratinocytes and serves as a ligand molecule of the CCR10 receptor expressed on the surfaces of lymphoid cells.

The amino acid sequence of CCL27 protein itself is publicly known in the art. The amino acid sequence of the protein can be obtained from a publicly known database such as SWISS-PROT (Accession number: Q9Y4X3).

CXCL9 is also referred to as "MIG", and is well-known to those skilled in the art as a chemokine which is induced by inflammatory stimulus such as IFN-γ or TNF-α in eosinophils, airway epithelial cells or the like.

The amino acid sequence of CXCL9 protein itself is publicly known in the art. The amino acid sequence of the protein can be obtained from a publicly known database such as National Center for Biotechnology Information (NCBI; Accession number: NM_002416).

In particular embodiments of the method of the present invention, an IL-25 marker may be added, in addition to at least one selected from CCL27 and CXCL9. IL-25 is a Pro-Th2 cytokine which is produced by Th2 cells, airway epithelial cells, mast cells or the like, and is well-known as a cytokine of the IL-17 family.

The amino acid sequence of IL-25 protein itself is publicly known in the art. The amino acid sequence of the protein can be obtained from a publicly known database such as SWISS-PROT (Accession number: Q9H293).

In the embodiments of the present method, "the amount of marker" means the amount of the marker protein in the blood sample. The measurement of the amount of marker includes a qualitative measurement for determining whether the marker protein is present in the blood sample.

The method used for the measurement of the amount of marker protein is not particularly limited as long as it is a protein assay based on antigen-antibody reaction, which assay is publicly known to those skilled in the art. Examples thereof include ELISA and Western blot assay. The ELISA assay is preferred. The antibody used for these assays is not particularly limited as long as it can specifically recognize each marker protein in the blood sample and bind thereto. Examples thereof may include monoclonal or polyclonal full-length antibodies or antibody fragments (Fab, F (ab')²). Such antibody production methods are publicly known to those skilled in the art. Alternatively, commercially available antibody products may be used.

In the measurement of the amount of the marker protein, a commercially available protein quantification kit which is publicly known to those skilled in the art, such as Duoset ELISA assay kit manufactured by R&D systems or ELISA development kit manufactured by Peprotech may be used.

The measured value of the amount of marker may be a measured value (raw data) itself or a value calculated based on the value, and it can be represented by any form or unit, such as concentration, intensity, level, mass (weight) or ratio.

A second step of the method provided herein includes obtaining an information related to eosinophilic airway inflammation in a subject based on the measured value obtained in the above measuring step (information obtaining step). Here, the information related to eosinophilic airway inflammation is not particularly limited as long as it is an information which may provide an indication of the diagnosis or prognosis of eosinophilic airway inflammation in the subject. An information showing the development or status of eosinophilic airway inflammation, or both, is preferred. Examples of the information include a probability that a subject has eosinophilic airway inflammation and a risk that the subject will develop eosinophilic airway inflammation. When the subject has eosinophilic airway inflammation, examples of the information related to eosinophilic airway inflammation include the subject's prognosis and the severity of eosinophilic airway inflammation.

In particular embodiments of the method of the present invention, when the measured values of the amount of a plurality of markers are used for analysis, the measured values of the amount of the markers are assigned to a multiple logistic regression model which is obtained from the measured values of the amount of the markers by substituting each of the measured values of the amount of the markers into a predetermined formula so that an information related to eosinophilic airway inflammation in a subject can be obtained. For example, when the measured values of CCL27 and CXCL9 are used for analysis, an information related to eosinophilic airway inflammation in a subject can be obtained based on the predictive values calculated from the measured values of CCL27and CXCL9 by the multiple logistic regression model. When the amount of IL-25 marker is further measured, an information related to eosinophilic airway inflammation in a subject can be obtained based on the predictive values calculated from the measured values of CCL27, CXCL9, and IL-25 by the multiple logistic regression model.

In particular embodiment of the methods of the present invention, an information related to eosinophilic airway inflammation in a subject can be obtained based on the results of a comparison of the measured value of the amount of the marker obtained in the measuring step with a predetermined threshold value for that same marker. In this instance, when the measured value obtained in the measuring step is higher than or equal to the predetermined threshold, an information to suggest that the subject has eosinophilic airway inflammation or an information that the subject is at high risk for eosinophilic airway inflammation can be obtained. When the subject has eosinophilic airway inflammation, an information that the subject's prognosis is poor or that the severity of eosinophilic airway inflammation is high can be obtained as the information related to eosinophilic airway inflammation. When the measured value obtained in the measuring step is a measured value of CCL27 and is higher than or equal to the predetermined threshold, an information determined as being positive for CCL27(+) or eosinophilic airway inflammation can be obtained. When the measured value obtained in the measuring step is a measured value of CXCL9 and is higher than or equal to the predetermined threshold, an information determined as being positive for CXCL9(+) or eosinophilic airway inflammation can be obtained.

On the contrary, when the measured value obtained in the measuring step is lower than the predetermined threshold, an information to suggest that the subject does not have eosinophilic airway inflammation or an information that the subject is at low risk for eosinophilic airway inflammation can be obtained. When the subject has eosinophilic airway inflammation, an information that the subject's prognosis is good or an information that the severity of eosinophilic airway inflammation is low can be obtained as the information related to eosinophilic airway inflammation. When the measured value obtained in the measuring step is a measured value of CCL27 and is lower than the predetermined threshold, an information determined as being negative for CCL27(-) or eosinophilic airway inflammation can be obtained. When the measured value obtained in the measuring step is a measured value of CXCL9 and is lower than the predetermined threshold, an information determined as being negative for CXCL9(-) or eosinophilic airway inflammation can be obtained.

When the measured values of the amount of a plurality of markers are used for analysis, an information related to eosinophilic airway inflammation in a subject can be obtained based on the results of comparison of each value calculated by assigning the measured values of the amount of the markers to a multiple logistic regression model which is obtained from the measured values of the amount of the markers with the predetermined threshold. For example, when the measured values of CCL27 and CXCL9 are used for analysis, and the predictive value calculated from the measured values of CCL27 and CXCL9 by the multiple logistic regression model is higher than or equal to the predetermined threshold, an information determined as being positive for eosinophilic airway inflammation can be obtained. On the contrary, when the predictive value calculated from the measured values of CCL27 and CXCL9 by the multiple logistic regression model is lower than the predetermined threshold, an information determined as being negative for eosinophilic airway inflammation can be obtained.

Note that the predetermined threshold is not particularly limited and it may be experimentally set by the accumulation of data for various blood samples. Alternatively, the predetermined threshold may be set as follows. First, the amount of marker in a blood sample of a subject unaffected with eosinophilic airway inflammation and a blood sample of a patient affected with eosinophilic airway inflammation is measured. On the basis of the obtained measurement results, the threshold is then determined from the range which is higher than the measured value in the blood sample of the subject unaffected with eosinophilic airway inflammation and is lower than the measured value in the blood sample of the patient affected with eosinophilic airway inflammation. Preferably, a value enabling the blood sample of the subject unaffected with eosinophilic airway inflammation to be distinguished from the blood sample of the patient affected with eosinophilic airway inflammation with a high degree of accuracy is determined as the threshold. Alternatively, a discrimination formula is generated by statistical analyses known in the art, and then the formula may be used to calculate the threshold from the measured values accumulated.

The scope of the present invention encompasses a marker for obtaining an information related to eosinophilic airway inflammation (hereinafter also simply referred to as "marker"). The marker is at least one selected from CCL27 and CXCL9.

Based on the measured value of the amount of marker in a blood sample collected from a subject, an information related to eosinophilic airway inflammation in the subject can be obtained. Note that the measurement of the amount of marker and the process of obtaining the information related to eosinophilic airway inflammation are the same as described above.

According to the present invention, there is provided an apparatus for obtaining an information related to eosinophilic airway inflammation (hereinafter, also simply referred to as "apparatus for obtaining an information") which includes
a measurement unit which obtains or is configured to obtain an information related to the amount of at least one marker selected from CCL27 and CXCL9 in a sample of a subject,
an information processing unit which obtains or is configured to obtain an information related to eosinophilic airway inflammation of the subject based on the information related to the amount of the marker obtained by the measurement unit, and
an output unit which outputs or is configured to output the information obtained by the information processing unit.

According to the present invention, there is provided a computer program for enabling a computer to perform a method as described herein, such as a method which includes steps of:
receiving an information related to the amount of at least one marker selected from CCL27 and CXCL9 in a sample of a subject;
obtaining an information related to eosinophilic airway inflammation of the subject based on the information related to the amount of the marker received in the receiving step; and
outputting the information obtained in the information obtaining step.

Note that in particular embodiments of the apparatus for obtaining an information and the computer program, the information related to the amount of marker may contain an information related to the amount of IL-25 marker.

Hereinafter, the apparatus for obtaining an information according to the present invention and computer programs will be described with reference to the drawings.

The apparatus for obtaining an information according to the present embodiment is an example of a preferred apparatus for performing the method to obtain an information according to the present invention. Note that the apparatus for obtaining an information according to the present embodiment is an apparatus which obtains an information by using the measured value of the amount calculated based on the luminescence intensity obtained by the protein assay as the information related to the amount of marker and comparing the measured value with a threshold of measured value.

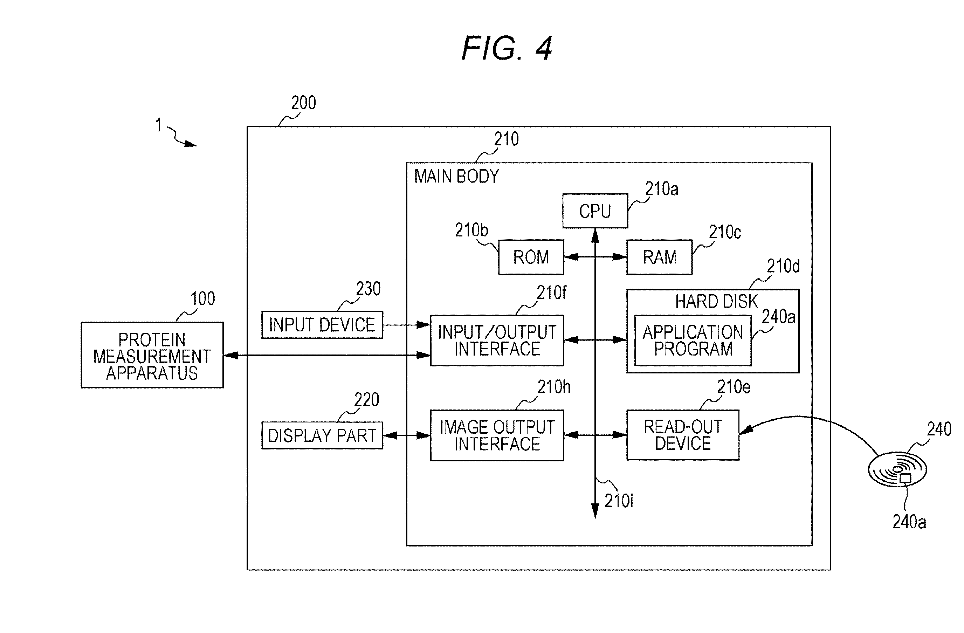

Fig. 4 is a block diagram showing a whole structure of an apparatus 1 for obtaining an information according to the first embodiment of the present invention.

The apparatus 1 for obtaining an information according to the present embodiment is configured by a protein measurement apparatus 100 (also referred to as "measurement unit 100") and an information processing apparatus 200 (also referred to as "computer 200") connected to the protein measurement apparatus 100.

The measurement unit 100 is configured by a protein measurement apparatus capable of performing a protein assay, such as a full automatic ELISA analyzer. The measurement unit 100 measures or is configured to measure an information related to the amount of marker observed when measuring the amount of marker in a blood sample, for example, the luminescence intensity.

The computer 200 is connected to the measurement unit 100. The computer 200 controls the operation of the measurement unit 100, and obtains an information of is configured to obtain information related to eosinophilic airway inflammation based on the data of the luminescence intensity measured by the measurement unit 100.

The computer 200 includes an information processing apparatus main body 210 (hereinafter also referred to as "information processing unit 210"), input device 230 which inputs necessary data or is configured to input the necessary date to the information processing unit 210, and display part (output unit) 220 which displays or is configured to display input/output data. The computer 200 includes an external recording medium 240, if necessary.

The computer 200 is mainly configured by the information processing unit 210, display part 220, and input device 230. The information processing unit 210 is data-communicably connected to a CPU 210a, ROM 210b, RAM 210c, hard disk 210d, read-out device 210e, input/output interface 210f, and image output interface 210h via a bus 210i.

The information processing unit 210 includes the CPU 210a, ROM 210b, RAM 210c, hard disk 210d, read-out device 210e, input/output interface 210f, image output interface 210h, and bus 210i.

In the information processing unit 210, the CPU 210a, ROM 210b, RAM 210c, hard disk 210d, read-out device 210e, input/output interface 210f, and image output interface 210h are respectively connected via the bus 210i so as to be capable of transmitting and receiving data.

The CPU 210a is capable of executing computer programs stored in the ROM 210b and computer programs loaded in the RAM 210c. A computer program which is executed by the CPU210a and data for the computer program which is executed by the CPU210a are recorded on the ROM210b. The RAM 210c is used when reading the computer programs recorded in the ROM 210b and stored on the hard disc 210d. The RAM 210c is used as a work area of the CPU 210a when these computer programs are executed.

The hard disc 210d contains various installed computer programs to be executed by the CPU 210a such as an operating system (OS) and application program, as well as data used in executing these computer programs.

The program installed on the hard disk 210d includes an application program 240a which realizes or is configured to execute the method to obtain an information related to eosinophilic airway inflammation and a program which controls the measurement unit 100. As data to be used for executing the program which realizes the method to obtain an information related to eosinophilic airway inflammation, threshold values or the like are stored on the hard disk 210d.

The read-out device 210e is configured by a flexible disc drive, CD-ROM drive, DVD-ROM drive or the like, and is capable of reading computer programs and data recorded on an external recording medium 240. The application program 240a may be recorded on the external recording medium 240, and on the ROM 210b or the RAM 210c formed in the information processing apparatus main body 210.

The configuration in which the CPU 210a reads the application program 240a from the external recording medium 240 and the application program 240a is installed on the hard disk 210d may be employed.

An operating system which provides a graphical user interface environment, for example, Windows (registered trademark) or the like, a product of Microsoft Corporation, USA, is installed on the hard disk 210d. In the following description, the application program 240a according to the above determination is assumed to be operating on the operating system.

The input/output interface 210f is configured by a serial interface such as a USB, IEEE1394 or RS-232C, parallel interface such as SCSI, IDE or IEEE1284, analog interface such as a D/A converter or A/D converter. The input/output interface 210f is connected to the input device 230 such as a keyboard and a mouse. The measurement unit 100 is connected to the input/output interface 210f. Thus, the information processing unit 210 can transmit and receive data to/from the measurement unit 100 via the input/output interface 210f.

The image output interface 210h is connected to the display part 220 which is configured by an LCD, CRT or the like, and outputs or is configured to output an image signal according to the image data received from the CPU 210a to the display part 220. The display part 220 displays or is configured to display the image (screen) according to the input image signal. The display part 220 outputs or is configured to output the determination result received from the CPU210a (described later).

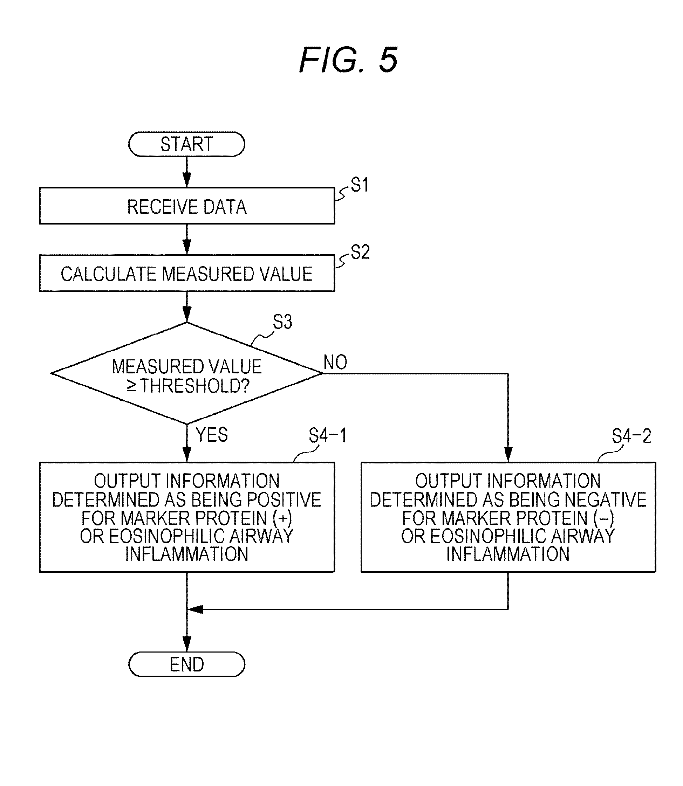

Hereinafter, an example of an operation flow of the application program 240a which is performed by the apparatus 1 for obtaining an information will be described with reference to Fig. 5.

First, the measurement unit 100 receives the data of luminescence intensity obtained by the protein assay via the input/output interface 210f (step S1).

In step S2, the CPU 210a then calculates the measured value of the amount of marker based on the data of luminescence intensity obtained by the protein assay.

In step S3, the CPU 210a compares the data of the threshold of luminescence intensity as the data of the application program 240a which has been pre-stored on the hard disk 210d with the data of luminescence intensity. In step S3, the CPU 210a determines whether the luminescence intensity is higher than the threshold of luminescence intensity.

When the luminescence intensity is not less than the threshold (Yes), the process is advanced to step S4-1. On the other hand, when the luminescence intensity is less than the threshold (No), the process is advanced to step S4-2.

In step S4-1, the CPU 210a stores the information determined as being positive for eosinophilic airway inflammation in the RAM 210c, and outputs the information on the display part 220 via the image output interface 210h. Specifically, when CCL27 is used as the marker, the CPU 210a outputs the information determined as being positive for CCL27 (+) or eosinophilic airway inflammation. When CXCL9 is used as the marker, the CPU 210a outputs the information determined as being positive for CXCL9 (+) or eosinophilic airway inflammation. An information that a subject has eosinophilic airway inflammation or is at high risk for eosinophilic airway inflammation may be output. Further, when the subject has eosinophilic airway inflammation, the information in which the subject's prognosis is poor or the severity of eosinophilic airway inflammation is high may be output.

In step S4-2, the CPU 210a stores the information determined as being negative for eosinophilic airway inflammation in the RAM 210c, and outputs the information on the display part 220 via the image output interface 210h. Specifically, when CCL27 is used as the marker, the CPU 210a outputs the information determined as being negative for CCL27 (-) or eosinophilic airway inflammation. When CXCL9 is used as the marker, the CPU 210a outputs the information determined as being negative for CXCL9 (+) or eosinophilic airway inflammation. An information that a subject does not have eosinophilic airway inflammation or is at low risk for eosinophilic airway inflammation may be output. Further, when the subject has eosinophilic airway inflammation, the information that the subject's prognosis is good or the severity of eosinophilic airway inflammation is low may be output.

In the present embodiment, the CPU 210a determines whether the luminescence intensity is higher than the threshold of luminescence intensity in step S3, however the present invention is not limited thereto. When the measured values of the amount of a plurality of markers are used for analysis, the CPU 210a may determine whether each value calculated by weighting the measured values of the amount of the markers according to importance by substituting each of the measured values of the amount of the markers into a predetermined formula is higher than the predetermined threshold. In this instance, the CPU 210a has pre-stored the predetermined threshold as the data of the application program 240a on the hard disk 210d.

Note that the data of the luminescence intensity obtained by the protein assay is obtained from the measurement unit 100 via the input/output interface 210f in the present embodiment, however the present invention is not limited thereto. A user inputs the data of the luminescence intensity obtained by the protein assay into the input device 230, and then the CPU 210a may obtain the input value as the luminescence intensity.

The present invention further comprises a computer system adapted to the method for obtaining an information related to eosinophilic airway inflammation. One embodiment of the computer system comprises a processor and a memory which is under control of the processor. In the memory the computer program mentioned above is stored. That is, the memory comprises software instructions adapted to enable the system to perform operations: receiving the amount of the marker; and obtaining the information related to eosinophilic airway inflammation.

Hereinafter, the present invention will be described in detail with reference to examples, however the present invention is not limited thereto.

### Examples

### Example 1 and Comparative Example 1

In order to examine the performance, when the markers CCL27 and CXCL9 were used singly, the following test was performed. For comparison, the same test was performed on Periostin as a well-known marker of eosinophilic airway inflammation as well as on Eotaxin for which the relation with eosinophil was suggested, and TARC (CCL17, CC Chemokine Ligand 17).

### (1) Preparation of samples

Serum samples of 39 patients with asthma recruited from the Sagamihara National Hospital were used. The sputum of these patients with asthma were used to perform the eosinophil test based on Hansel stain method. As a result, the sputum eosinophils were detected in 33 patients (sputum eosinophil-positive), whereas the sputum eosinophils were not detected in 6 patients (sputum eosinophil-negative).

### (2) ELISA assay

In order to measure the amount of each marker using the serum samples obtained in the above process, ELISA assay was performed as follows.

### (2-1) CCL27

An ELISA assay kit (Duoset (DY376), manufactured by R&D systems) was used.

One hundred (100) µL of antibody diluting liquid (4 µg/mL of solid phase antibody, 0.05% NaN₃, PBS pH7.4) was first added to a 96-well ELISA plate, and then the antibody was solid-phased at 4°C overnight. The resultant solid phase was washed 3 times with a washing buffer (0.05% Tween20, 0.05% NaN₃, PBS pH7.4). The solid phase was blocked with 250 µL of blocking buffer (1% BSA, 0.5% Casein, 0.05% Tween20, 0.05% NaN₃, PBS pH7.4) at room temperature for not less than 2 hours. One hundred (100) µL of serum 4-fold diluted with an ELISA dilution buffer (1% BSA, 0.5% Casein, 0.05% NaN₃, PBS pH7.4) was added to the solid phase, followed by reaction at 4°C overnight. The resultant mixture was washed 3 times with a washing buffer and 100 µL of labeled antibody solution (0.075 µg/mL of labeled antibody, 1% BSA, 0.5% Casein, 0.05% NaN₃, PBS pH7.4) was added thereto. The resultant mixture was reacted at room temperature for 2 hours, followed by washing 3 times with a washing buffer. One hundred (100) µL of alkaline phosphatase-labeled streptavidin solution (1:1000 dilution, Streptavidin-Alkaline Phosphatase [R&D systems AR001], 1% BSA, 0.5% Casein, 0.05% NaN₃, PBS pH7.4) was added to the mixture, followed by reaction for 20 minutes. The resultant mixture was washed 3 times with a washing buffer, and then washed once with 250 µL of ALP activated buffer (20 mM Tris, 10 mM MgCl, pH9.8). One hundred (100) µL of substrate (CDP-Star Ready-to-use Sapphire II Tropix) was added to the resultant mixture, and the luminescence signals were detected with a chemiluminescent detector (FLUO star OPTIMA BMG LabTech).

### (2-2) CXCL9

An ELISA assay kit (Duoset (DY392), manufactured by R&D systems) was used. One hundred (100) µL of antibody diluting liquid (1 µg/mL of solid phase antibody, PBS pH7.4) was first added to a 96-well ELISA plate, and then the antibody was solid-phased at 4°C overnight. The resultant solid phase was washed 3 times with a washing buffer, and the solid phase was blocked with 250 µL of blocking buffer at room temperature for not less than 2 hours. One hundred (100) µL of serum 4-fold diluted with an ELISA dilution buffer (1% BSA, 0.5% Casein, 0.05% NaN₃, PBS pH7.4) was added to the solid phase, followed by reaction at 4°C overnight. The resultant mixture was washed 3 times with a washing buffer and 100 µL of labeled antibody solution (0.2 µg/mL of labeled antibody, 1% BSA, 0.5% Casein, 0.05% NaN₃, PBS pH7.4) was added thereto. The resultant mixture was reacted at room temperature for 2 hours, followed by washing 3 times with a washing buffer. One hundred (100) µL of alkaline phosphatase-labeled streptavidin solution (1:1000 dilution, Streptavidin-Alkaline Phosphatase, 1% BSA, 0.05% NaN₃, PBS pH7.4) was added to the mixture, followed by reaction for 20 minutes. The resultant mixture was washed 3 times with a washing buffer and washed with 250 µL of ALP activated buffer. A substrate was added thereto, and the luminescence signals were detected with a 100 µL chemiluminescent detector.

### (2-3) Periostin

An ELISA assay kit (Duoset (DY3548), manufactured by R&D systems) was used.

Fifty (50) µL of antibody diluting liquid (1 µg/mL of solid phase antibody, PBS pH7.4) was first added to a 384-well ELISA plate, and then the antibody was solid-phased at 4°C overnight. The resultant solid phase was washed 3 times with a washing buffer. The solid phase was blocked with 100 µL of blocking buffer (1% BSA, 0.5% Casein, 0.05% Tween20, 0.05% NaN₃, PBS pH7.4) at room temperature for not less than 2 hours. Fifty (50) µL of serum 100-fold diluted with an ELISA dilution buffer (1% BSA, 0.5% Casein, 0.05% NaN₃, PBS pH7.4) was added to the solid phase, followed by reaction at 4°C overnight. The resultant mixture was washed 3 times with a washing buffer and 50 µL of labeled antibody solution (2 µg/mL of labeled antibody, 1% BSA, 0.5% Casein, 0.05% NaN₃, PBS PH7.4) was added thereto. The resultant mixture was reacted at room temperature for 2 hours, followed by washing 3 times with a washing buffer. Fifty (50) µL of alkaline phosphatase-labeled streptavidin solution (1:1000 dilution, Streptavidin-Alkaline Phosphatase, 1% BSA, 0.5% Casein, 0.05% NaN₃, PBS pH7.4) was added to the mixture, followed by reaction for 20 minutes. The resultant mixture was washed 3 times with a washing buffer and washed with 100 µL of ALP activated buffer. Fifty (50) µL of substrate was added thereto, and the luminescence signals were detected with a chemiluminescent detector (FLUO star OPTIMA BMG LABTECH).

### (2-4) Eotaxin

An ELISA assay kit (Duoset (DY320), manufactured by R&D systems) was used.

One hundred (100) µL of antibody diluting liquid (2 µg/mL of solid phase antibody, PBS pH7.4) was first added to a 96-well ELISA plate, and then the antibody was solid-phased at 4°C overnight. The resultant solid phase was washed 3 times with a washing buffer, and the solid phase was blocked with 250 µL of blocking buffer at room temperature for not less than 2 hours. One hundred (100) µL of serum 4-fold diluted with an ELISA dilution buffer (1% BSA, 0.5% Casein, 0.05% NaN₃, PBS pH7.4) was added to the solid phase, followed by reaction at 4°C overnight. The resultant mixture was washed 3 times with a washing buffer and 100 µL of labeled antibody solution (0.1 µg/mL of labeled antibody, 1% BSA, 0.5% Casein, 0.05% NaN₃, PBS pH7.4) was added thereto. The resultant mixture was reacted at room temperature for 2 hours, followed by washing 3 times with a washing buffer. One hundred (100) µL of alkaline phosphatase-labeled streptavidin solution (1:1000 dilution, Streptavidin-Alkaline Phosphatase, 1% BSA, 0.5% Casein, 0.05% NaN₃, PBS pH7.4) was added to the mixture, followed by reaction for 20 minutes. The resultant mixture was washed 3 times with a washing buffer and washed with 250 µL of ALP activated buffer. One hundred (100) µL of substrate was added thereto, and the luminescence signals were detected with a chemiluminescent detector.

### (2-5) TARC

An ELISA assay kit (Duoset (DY364), manufactured by R&D systems) was used.

One hundred (100) µL of antibody diluting liquid (2 µg/mL of solid phase antibody, PBS pH7.4) was first added to a 96-well ELISA plate, and then the antibody was solid-phased at 4°C overnight. The resultant solid phase was washed 3 times with a washing buffer, and the solid phase was blocked with 250 µL of blocking buffer at room temperature for not less than 2 hours. One hundred (100) µL of serum 4-fold diluted with an ELISA dilution buffer (1% BSA, 0.05% NaN₃, PBS PH7.4) was added to the solid phase, followed by reaction at 4°C overnight. The resultant mixture was washed 3 times with a washing buffer and 100 µL of labeled antibody solution (0.1 µg/mL of labeled antibody, 1% BSA, 0.05% NaN₃, PBS pH7.4) was added thereto. The resultant mixture was reacted at room temperature for 2 hours, followed by washing 3 times with a washing buffer. One hundred (100) µL of alkaline phosphatase-labeled streptavidin solution (1:1000 dilution, Streptavidin-Alkaline Phosphatase, 1% BSA, 0.05% NaN₃, PBS pH7.4) was added to the mixture, followed by reaction for 20 minutes. The resultant mixture was washed 3 times with a washing buffer and washed with 250 µL of ALP activated buffer. One hundred (100) µL of substrate was added thereto, and the luminescence signals were detected with a chemiluminescent detector.

### (3) Analyses of ELISA assay results

As for each marker, a calibration curve was created from the luminescence signal (RLU) of the known concentration standard by 4-parameter fit. The luminescence signal (RLU) obtained from each specimen was applied to the calibration curve, and the marker concentration in serum was calculated. An analysis software; MARS (BMG LABTECH) was used for analysis.

### (4) Statistical analyses

Statistical analyses were performed using StatFlex Version 6.0 (YUMIT).

### (4-1) Significant difference test

According to sputum eosinophil-positive or -negative, the patient group was divided into two groups, and differences in concentrations among markers in serum were examined by Mann-Whitney test. The test results are summarized in Table 1-1.

**[Table 1-1]**

| Marker | p value |
|---|---|
| CCL27 | 0.0195 |
| CXCL9 | 0.0056 |
| Periostin | 0.0798 |
| Eotaxin | 0.1857 |
| TARC | 0.7703 |

### (4-2) Calculation of sensitivity and specificity

The ROC curve was found, the cutoff value of sputum eosinophil-positive or -negative cutoff was set, and the sensitivity and specificity were calculated.

The cutoff value was set to a point closest to the points where the sensitivity and 1-specificity are 1 and 0, respectively in the ROC curve (indicated by an arrow in each drawing).

### (4-3) Comparison of ROC curves

The areas under the curve (AUC) of the ROC curves among respective items were measured by the Hanley method.

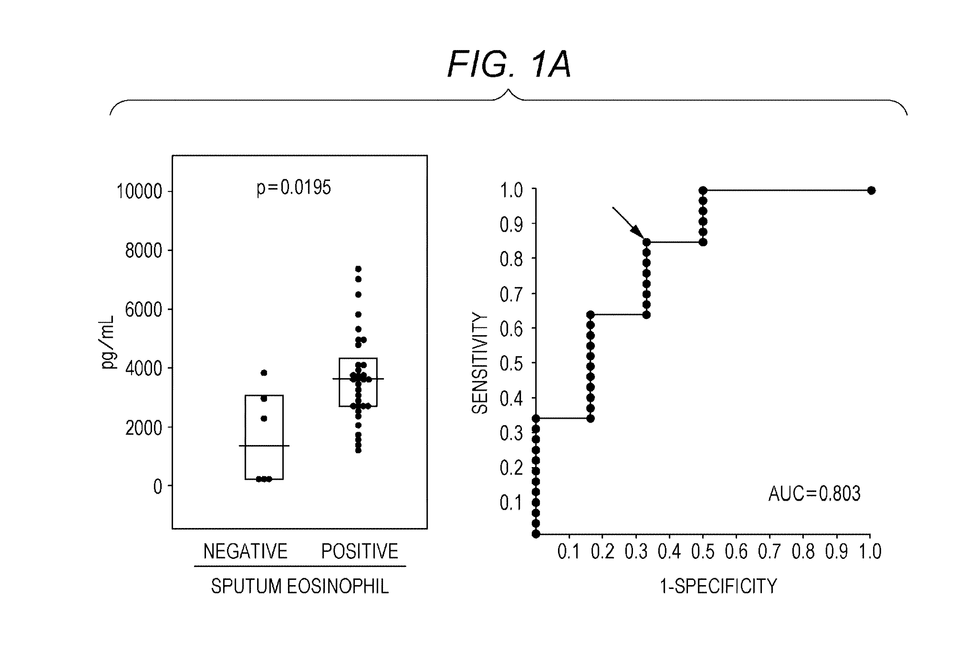

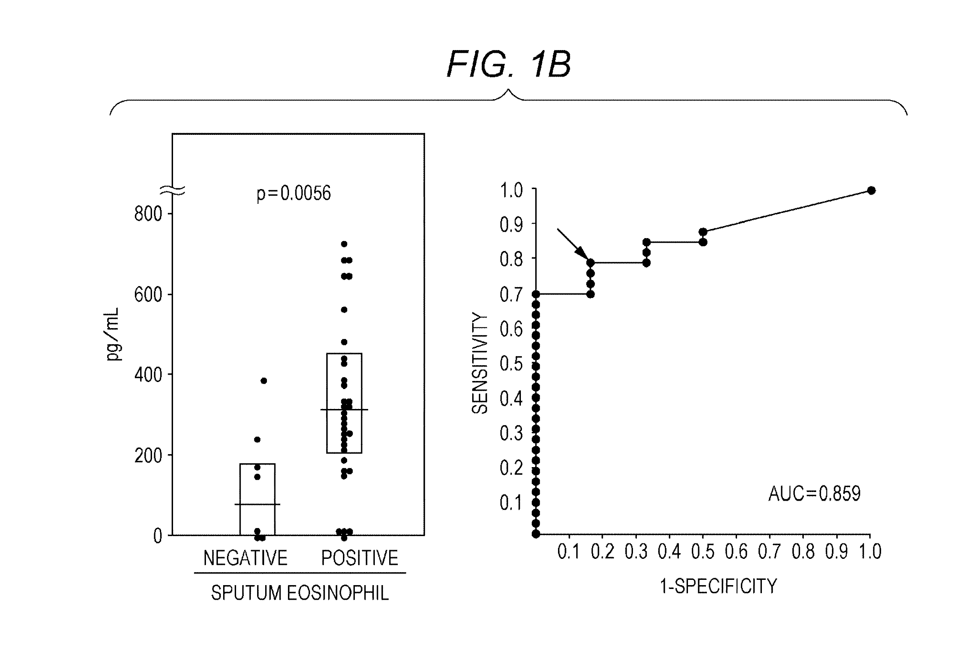

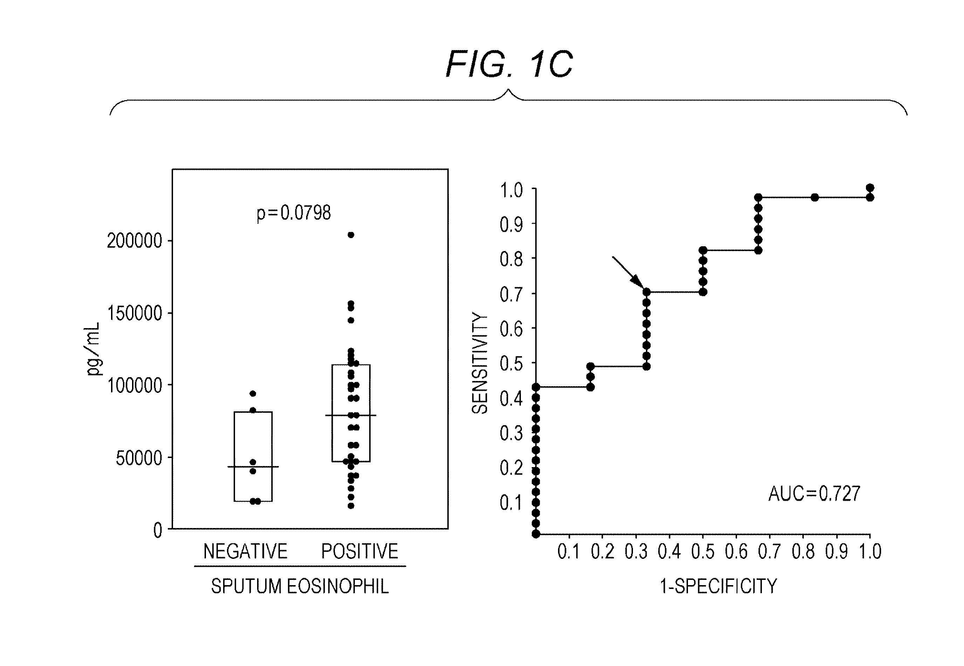

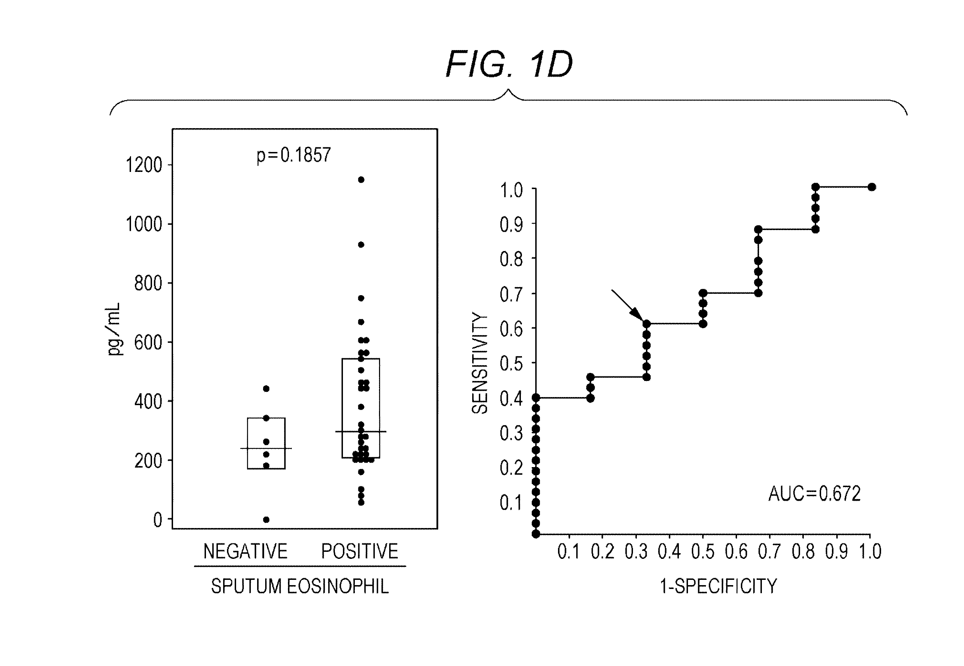

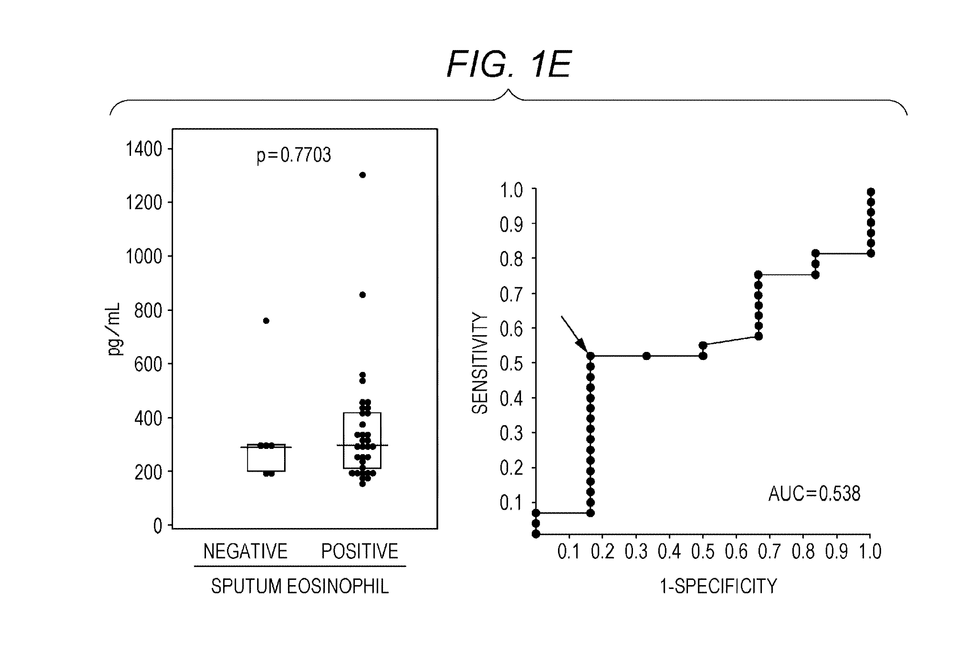

The analysis results obtained regarding CCL27 (Fig. 1A) and CXCL9 (Fig. 1B) are shown as Example 1. The analysis results obtained regarding Periostin (Fig. 1C), Eotaxin (Fig. 1D), and TARC (Fig. 1E) are shown as Comparative Example 1. Further, the analysis results are summarized in Table 1-2 below.

**[Table 1-2]**

| Marker | Cutoff value (pg/mL) | Sensitivity | Specificity | AUC |
|---|---|---|---|---|
| CCL27 | 2273 | 0.85 | 0.67 | 0.803 |
| CXCL9 | 175 | 0.79 | 0.83 | 0.859 |
| Periostin | 49474 | 0.67 | 0.67 | 0.727 |
| Eotaxin | 247 | 0.61 | 0.50 | 0.672 |
| TARC | 296 | 0.52 | 0.83 | 0.538 |

Table 1-1 shows that, regarding Periostin, Eotaxin, and TARC, no significant difference in marker concentration between sputum eosinophil-positive and -negative patients was observed, whereas, regarding CCL27 and CXCL9, a significant difference in marker concentration between sputum eosinophil-positive and -negative patients was observed. This result shows that CCL27 and CXCL9 are markers capable of discriminating between sputum eosinophil-positive and -negative patients with asthma.

From the results of Table1-2, it is clear that CCL27 and CXCL9 are markers having a performance superior to Periostin, Eotaxin, and TARC, from the viewpoint of sensitivity and specificity.

### Example 2 and Comparative Example 2

In Example 2, in order to examine the performance as primary screening markers for eosinophilic airway inflammation regarding CCL27 and CXCL9, the inventors measured the marker concentration in healthy adults without asthma, compared the result with the marker concentration obtained in Example 1, and analyzed them. Specifically, the ELISA-based assay of the marker concentration was performed in the same manner as Example 1 using serum samples from 12 healthy volunteers as specimens of the healthy adults without asthma. The measurement results were combined with the data of the marker concentration obtained from 39 specimens of the serum samples used in Example 1, and statistical analyses were performed in the same manner as Example 1. Note that the healthy adults without asthma were treated as the sputum eosinophil-negative group.

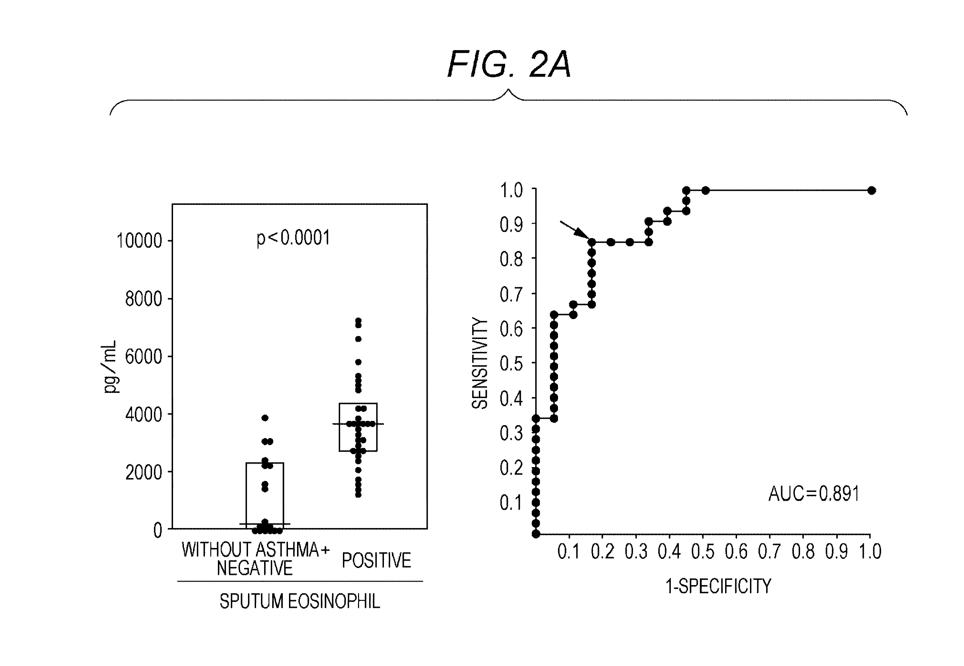

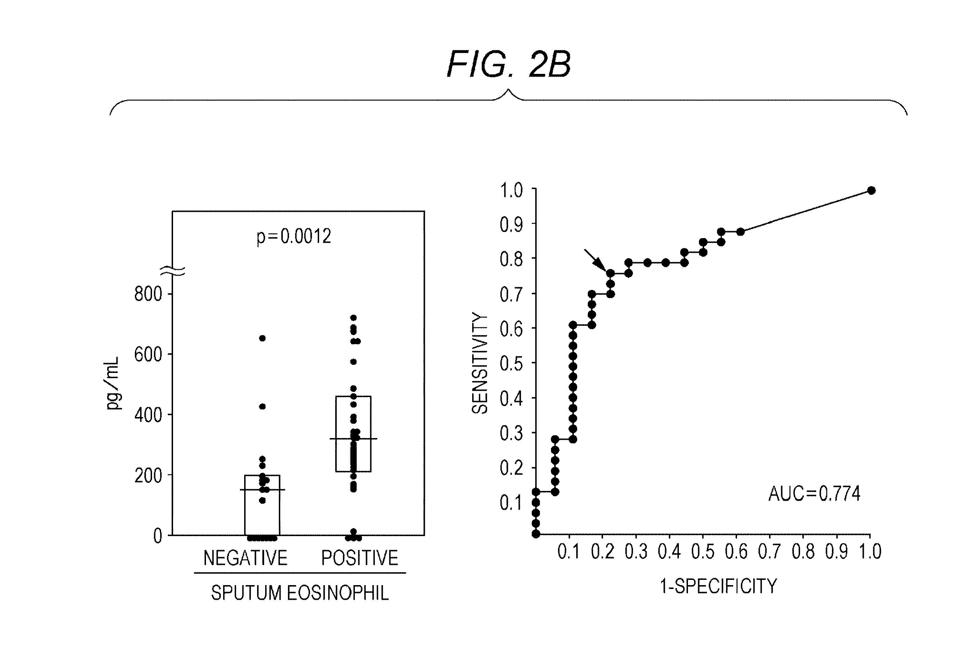

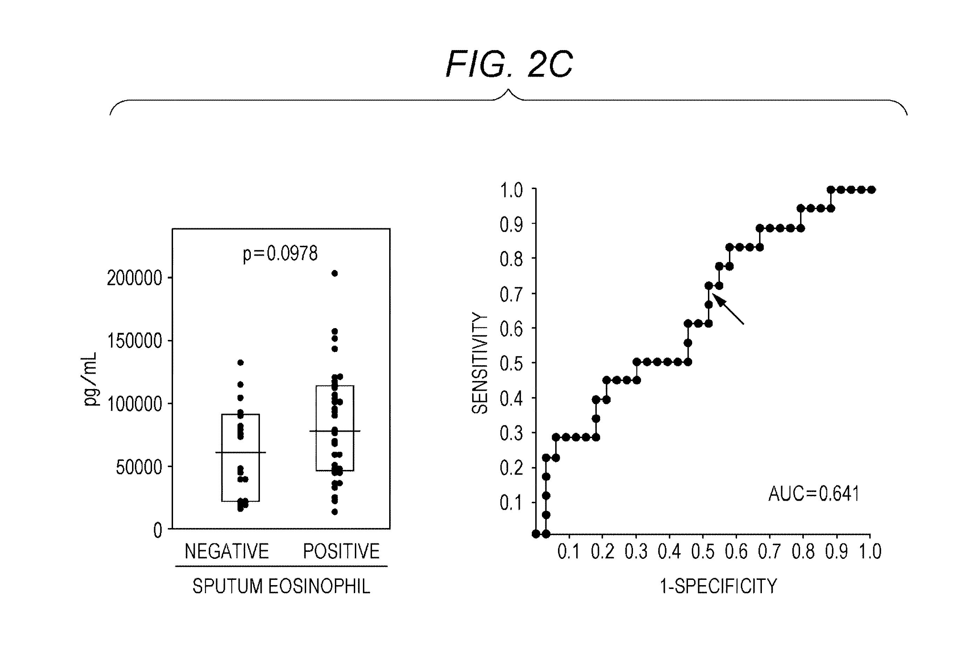

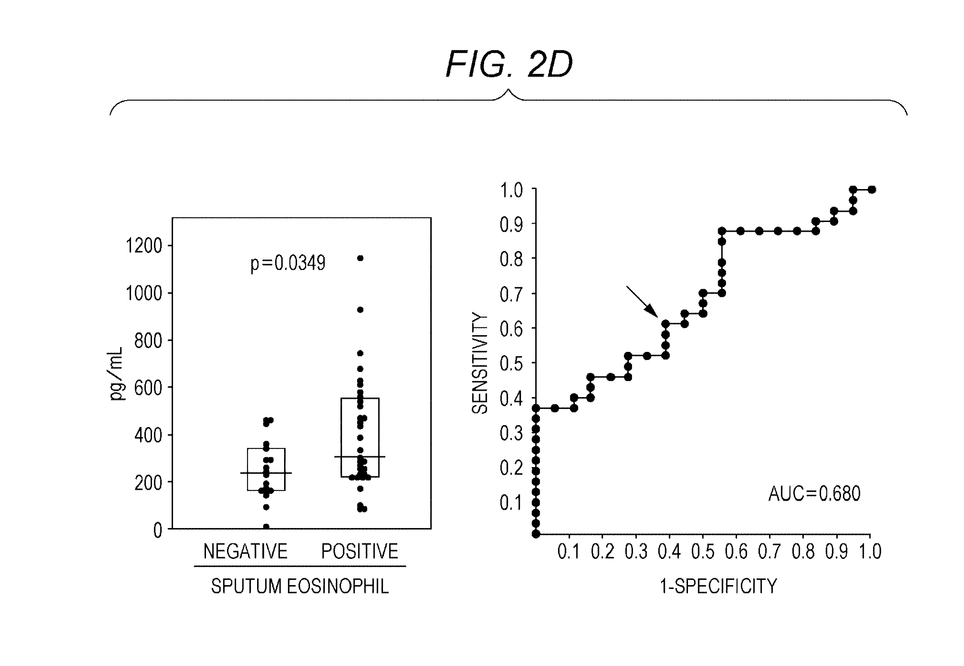

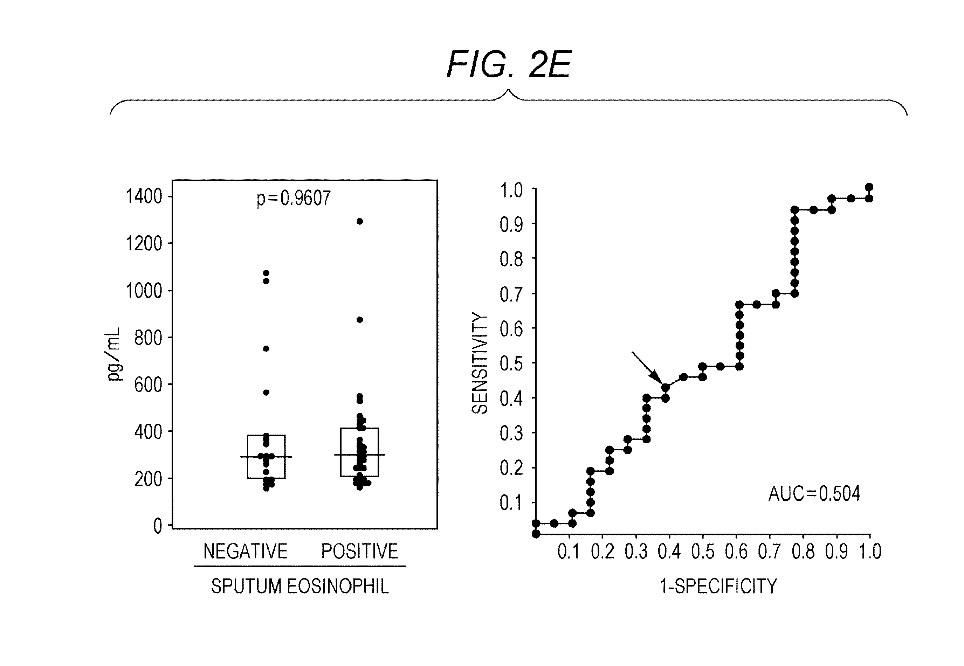

The analysis results obtained regarding CCL27 (Fig. 2A) and CXCL9 (Fig. 2B) are shown as Example 2. The analysis results obtained regarding Periostin (Fig. 2C), Eotaxin (Fig. 2D), and TARC (Fig. 2E) are shown as Comparative Example 2. Further, the analysis results are summarized in Tables 2-1 and 2-2 below.

**[Table 2-1]**

| Marker | p value |
|---|---|
| CCL27 | Less than 0.0001 |
| CXCL9 | 0.0012 |
| Periostin | 0.0978 |
| Eotaxin | 0.0349 |
| TARC | 0.9607 |

**[Table 2-2]**

| Marker | Cutoff value (pg/mL) | Sensitivity | Specificity | AUC |
|---|---|---|---|---|
| CCL27 | 2385 | 0.85 | 0.83 | 0.891 |
| CXCL9 | 199 | 0.76 | 0.78 | 0.774 |
| Periostin | 46926 | 0.73 | 0.44 | 0.641 |
| Eotaxin | 255 | 0.61 | 0.61 | 0.680 |
| TARC | 284 | 0.42 | 0.61 | 0.504 |

Table 2-1 shows that, regarding Periostin and TARC, no significant difference in marker concentration between sputum eosinophil-positive and -negative patients was observed, whereas, regarding CCL27, CXCL9, and Eotaxin, a significant difference in marker concentration between a sputum eosinophil-positive group (sputum eosinophil-positive patients) and a sputum eosinophil-negative group (sputum eosinophil-negative patients and healthy adults without asthma) was observed. This result shows that CCL27, CXCL9, and Eotaxin are markers capable of discriminating between the sputum eosinophil-positive and -negative groups.

From the results of Table 2-2, it is clear that CCL27 and CXCL9 have performance superior to Periostin, Eotaxin, and TARC as primary screening markers for eosinophilic airway inflammation, from the viewpoint of sensitivity and specificity. When the healthy adults are included, particularly, the specificity of CCL27 is significantly improved as compared with when the healthy adults are not included (Example 1, Fig. 1A). As a result, the AUCs show very favorable values (Table 2 and Fig. 2A). The above results indicate that CCL27 can be used as a marker having particularly excellent performance in primary screening for eosinophilic airway inflammation in the healthy adults and patients with asthma.

Considering all the results of Examples 1 and 2 together, CCL27 and CXCL9 can be used as markers for eosinophilic airway inflammation which have performance superior to eosinophil-related proteins and well-known markers for eosinophilic airway inflammation. Further, CCL27 is considered to have favorable performance, particularly as a marker for selecting patients suspected of being affected with eosinophilic airway inflammation from the general population including subjects unaffected with eosinophilic airway inflammation. On the other hand, CXCL9 is considered to have favorable performance, particularly as a marker for discriminating patients affected with eosinophilic airway inflammation from patients unaffected with eosinophilic airway inflammation in patients affected with asthma.

### Example 3

In Example 3, the inventors examined whether excellent discrimination performance was obtained by a combination of markers CCL27 and CXCL9 which independently exhibited favorable discrimination performance. They further examined whether excellent discrimination performance was given by combining the markers CCL27 and CXCL9 with IL-25.

As for the concentration of IL-25, an ELISA assay was performed on the same serum sample as that in which the data of the marker concentration was obtained in Example 1. Specifically, it was performed as follows.

An ELISA development kit (900-K234), manufactured by Peprotech was used for the ELISA assay.

One hundred (100) µL of antibody diluting liquid (1 µg/mL of solid phase antibody, PBS pH 12.0) was first added to an ELISA plate, and then the antibody was solid-phased at 4°C overnight. The resultant solid phase was washed 3 times with a washing buffer, and the solid phase was blocked with 250 µL of blocking buffer at room temperature for not less than 2 hours. One hundred (100) µL of serum 4-fold diluted with an ELISA dilution buffer (1% BSA, 0.5% Casein, 0.05% NaN₃, PBS pH7.4) was added to the solid phase, followed by reaction at 4°C overnight. The resultant mixture was washed 3 times with a washing buffer and 100 µL of labeled antibody solution (0.25 µg/mL of labeled antibody, 1% BSA, 0.5% Casein, 0.05% NaN₃, PBS) was added thereto. The resultant mixture was reacted at room temperature for 2 hours, followed by washing 3 times with a washing buffer. One hundred (100) µL of alkaline phosphatase-labeled streptavidin solution (1:1000 dilution, Streptavidin-Alkaline Phosphatase, 1% BSA, 0.05% NaN₃, PBS pH7.4) was added to the mixture, followed by reaction for 20 minutes. The resultant mixture was washed 3 times with a washing buffer and washed with 250 µL of ALP activated buffer. One hundred (100) µL of substrate was added thereto, and the luminescence signals were detected with a chemiluminescent detector.

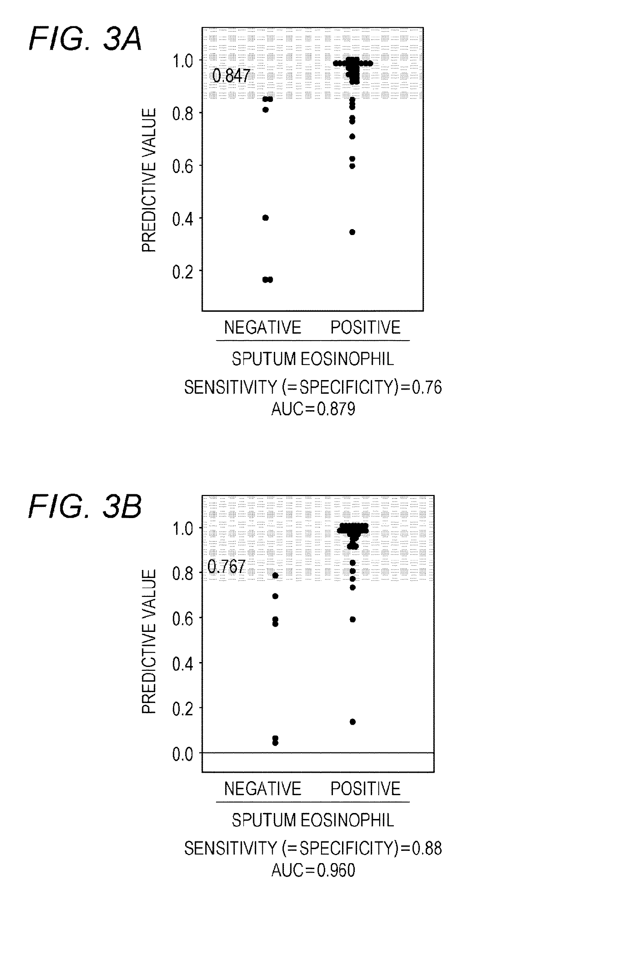

Then, a multiple logistic regression model was created using measurement results of sputum eosinophils (positive/negative) as objective variables and concentrations of CCL27 and CXCL9 in serum as independent variables. The predictive values of the measurement results of sputum eosinophils were obtained by assigning each of the measured values of the amount of the markers to the multiple logistic regression model (variable designation). The predictive values for the marker concentration obtained from the serum samples were calculated. It is found that most of the predictive values calculated for the sputum eosinophil-positive patients are centered around 1.0, whereas the predictive values calculated for the sputum eosinophil-negative patients are distributed around 0 to 0.8 (Fig. 3A). Then, the ROC analysis was performed based on the calculated predictive values. The cutoff value was set at a point where the sensitivity coincides with the specificity, and the AUC value was calculated. As a result, the cutoff value was about 0.847 (the sensitivity and specificity were 0.76), and the AUC value was about 0.879. The set cutoff values are shown in Fig. 3A. It is found that sputum eosinophil-positive patients with asthma can be discriminated from sputum eosinophil-negative patients with asthma with a high degree of accuracy by using the cutoff values.

Further, a multiple logistic regression model was created using measurement results of sputum eosinophils (positive/negative) as objective variables and concentrations of CCL27, CXCL9, and IL-25 in serum as independent variables. The predictive values of the measurement results of sputum eosinophils were obtained by assigning each of the measured values of the amount of the markers to the multiple logistic regression model (variable designation). The predictive values for the marker concentration obtained from the serum samples were calculated. It is found that most of the predictive values calculated for the sputum eosinophil-positive patients are centered around 1.0, whereas the predictive values calculated for the sputum eosinophil-negative patients are distributed around 0.2 to 0.85 (Fig. 3B). Then, the ROC analysis was performed based on the calculated predictive values. The cutoff value was set at a point where the sensitivity coincides with the specificity, and the AUC value was calculated. As a result, the cutoff value was about 0.767 (the sensitivity and specificity were 0.88), and the AUC value was about 0.960. The determined cutoff values are shown in Fig. 3B. It is found that sputum eosinophil-positive patients with asthma can be discriminated from sputum eosinophil-negative patients with asthma with a high degree of accuracy by using the cutoff values.

From the comparison results of Figs.3A and 1B, it is suggested that the combination of markers CCL27 and CXCL9 (Fig. 3A) shows excellent performance for discriminating sputum eosinophil-positive patients with asthma from sputum eosinophil-negative patients with asthma, compared to when these markers are used singly (Example1).

Further, it is suggested that when IL-25 is combined (Fig. 3B), in addition to the marker, excellent discrimination performance is exhibited, compared to when CCL27 is combined with CXCL9 (Fig. 3A).

## Claims

1. A method of obtaining an information related to eosinophilic airway inflammation in a subject, **characterized in that** the method comprises steps of:
measuring an amount of at least one marker selected from CXCL9 and CCL27 in a blood sample in a subject; and
obtaining an information related to eosinophilic airway inflammation in the subject based on the resulting measured value.

2. The method according to claim 1, further comprising comparing the measured amount with a predetermined threshold, and
the information related to eosinophilic airway inflammation in the subject is obtained based on the comparison result.

3. The method according to claim 1 or 2, wherein
the information related to eosinophilic airway inflammation in the subject is an information indicating that the subject has eosinophilic airway inflammation or an information to suggest that the subject does not have eosinophilic airway inflammation.

4. The method according to any of claims 1 to 3, wherein
the amounts of markers CCL27 and CXCL9 in the blood sample of the subject are measured in the measuring step, and
the information related to eosinophilic airway inflammation in the subject is obtained based on the measured amounts.

5. The method according to claim 4, further comprising
obtaining a predictive value from a multiple logistic regression model based on the measured amounts of CCL27 and CXCL9; and comparing the predictive value to a predetermined threshold, and
wherein the information related to eosinophilic airway inflammation in the subject is obtained based on the comparison result.

6. The method according to any of claims 1 to 5, further comprising measuring an amount of IL-25 marker in the blood sample, and
the information related to eosinophilic airway inflammation in the subject is obtained based on the amount of at least one marker selected from the group consisting of CCL27 and CXCL9 and the amount of IL-25 marker.

7. The method according to any of claims 1 to 6, further comprising measuring an amount of IL-25 in the blood sample,
wherein the amounts of markers CXCL9 and CCL27 in the blood sample are measured in the measuring step,
the information related to eosinophilic airway inflammation in the subject is obtained based on the measured amounts of CCL27, CXCL9, and IL-25.

8. The method according to any of claims 1 to 7, wherein the blood sample is whole blood, blood serum or blood plasma.

9. An apparatus for obtaining an information related to eosinophilic airway inflammation, comprising:
a measurement unit which is configured to obtain an information related to an amount of at least one marker selected from CXCL9 and CCL27 in a blood sample of a subject;
an information processing unit which is configured to obtain an information related to eosinophilic airway inflammation of the subject based on the information related to the amount of the marker obtained by the measurement unit; and
an output unit which is configured to output the information obtained by the information processing unit.
